# DEMANDE DE BREVET EUROPEEN

(11) **EP 3 182 381 A1**
(43) Date de publication de la demande: **21.06.2017**
(21) Numéro de dépôt: 15201308.2
(22) Date de dépôt: 18.12.2015
(51) Int. Cl.: G07C 1/24, A63B 71/06

(54) **SYSTEME DE MESURE AVEC CORRECTION DU TEMPS D'ARRIVEE D'UN ATHLETE DANS UNE COURSE**

(71) Demandeur: Swiss Timing Ltd., 2606 Corgémont (CH)
(72) Inventeur: Galli, Reto, 3053 Münchenbuchsee (CH); Kollega, Alexander, 04275 Leipzig (DE); Massé, Fabien, 1004 Lausanne (CH)
(74) Mandataire: Giraud, Eric

(57) **Abrégé**

Le système de mesure permet de corriger un temps d'arrivée d'un athlète (20) dans une course à pieds. Le système comprend un module à transpondeur (1) personnalisé et placé sur une partie haute du corps de l'athlète et une station de base (10). Le module à transpondeur comprend une unité de réception (3) de signaux, une unité de traitement (4) de données, de mesures ou de commandes, une unité de transmission (5) de signaux de données et/ou de mesures et/ou de commandes, et un capteur de mouvement (7) pour fournir des signaux de mesure à l'unité de traitement. Le capteur de mouvement peut être un gyroscope (7) relié à l'unité de traitement dans le module à transpondeur et configuré pour déterminer un angle d'inclinaison du corps de l'athlète par rapport à une direction verticale lors du passage de la ligne d'arrivée (30) pour une correction du temps d'arrivée de course.

## Description

### Domaine de l'invention

L'invention concerne un système de mesure avec correction du temps d'arrivée d'un athlète dans une course à pieds au moyen d'un module à transpondeur adapté à l'athlète et porté de préférence sur une partie haute du corps de l'athlète durant la course.

L'invention concerne également un procédé de mesure avec correction du temps d'arrivée d'un athlète dans une course à pieds pour la mise en action du système de mesure.

### Arrière-plan de l'invention

Lors d'une compétition sportive comme une course de sprint en athlétisme, chaque athlète part depuis un starting-block. Chaque athlète est souvent équipé d'un module à transpondeur, qui est intégré dans le dossard à numéro. Le chronométrage ou le temps de course non officiel de l'athlète est pris depuis la lecture du module à transpondeur, qui apparaît lorsque ledit module à transpondeur passe la ligne d'arrivée. Après cela, le temps officiel est jugé suite à l'image photofinish prise en tenant compte des règles IAAF 165.2.

Il est à noter que ce temps doit être pris au moment, où n'importe quelle partie du torse, atteint le plan vertical à proximité du bord de la ligne d'arrivée. Ce temps officiel est normalement disponible environ entre 10 et 20 s après le temps non officiel, ce qui constitue une longue période pour des spectateurs visionnant ladite course.

Si un athlète passe la ligne d'arrivée en étant dans une position droite, le module à transpondeur est dans une position, qui correspond tout à fait à la position pour juger le temps sur l'image photofinish. Ainsi, l'erreur entre le temps non officiel du module à transpondeur et le temps officiel tiré de l'image photofinish diffère seulement en fonction de l'imprécision de la détection du module à transpondeur. Cependant, beaucoup d'athlètes se plient vers l'avant, lorsqu'ils passent la ligne d'arrivée pour obtenir un avantage dans le jugement par image photofinish. Dans ce cas, l'athlète est jugé sur les épaules vers l'avant avec l'image photofinish, alors que le module à transpondeur passe la ligne d'arrivée plus de 10 cm derrière ce point d'arrivée. Ainsi, la différence entre les temps non officiel du module à transpondeur et officiel peut être assez grande. De plus, dans le cas d'une arrivée serrée de plusieurs athlètes, le classement de chacun peut aussi être erroné.

Selon l'état de la technique, et en fonction de la différence de temps susmentionnée entre le temps du transpondeur et le temps de l'image photofinish, le classement non officiel est publié uniquement quand la différence du temps du module à transpondeur de deux athlètes est plus grande que 0.1 s. Si la différence est plus faible, aucun classement n'est montré aux spectateurs, jusqu'à ce que les temps officiels de l'image photofinish soient disponibles.

### Résumé de l'invention

L'invention a donc pour but de pallier les inconvénients de l'état de la technique susmentionné en proposant un système de mesure avec correction du temps d'arrivée d'un athlète dans une course à pieds au moyen d'un module à transpondeur adapté à l'athlète et porté sur une partie haute du corps de l'athlète durant la course.

A cet effet, l'invention concerne un système de mesure avec correction du temps d'arrivée d'un athlète dans une course, qui comprend les caractéristiques définies dans la revendication indépendante 1.

Des formes d'exécution particulières du système de mesure avec correction du temps d'arrivée d'un athlète dans une course sur piste sont définies dans les revendications dépendantes 2 à 9.

Un avantage du système de mesure avec correction du temps d'arrivée réside dans le fait qu'avec le module à transpondeur placé sur une partie haute du corps de l'athlète, il est possible de corriger le temps d'arrivée d'un athlète suite au passage du module à transpondeur sur la ligne d'arrivée. Le module à transpondeur comprend au moins un capteur de mouvement capable de fournir une information sur un angle d'inclinaison de la partie haute du corps de l'athlète lors du passage de la ligne d'arrivée. Avec l'information fournie par le module à transpondeur sur l'instant de passage de la ligne d'arrivée et l'angle d'inclinaison déterminé, cela permet d'estimer le temps d'arrivée du module à transpondeur. Une correction du temps d'arrivée non officiel sur la base du passage du module à transpondeur sur la ligne d'arrivée peut ainsi être calculée.

Avantageusement, le module à transpondeur comprend au moins un capteur de mouvement pour mesurer l'angle l'inclinaison de la partie haute du corps de l'athlète. Ce capteur de mouvement peut être un capteur de mouvement inertiel. Il peut être un gyroscope relié à une unité de traitement capable d'effectuer des calculs sur la base des mesures du gyroscope pour transmettre l'information d'angle d'inclinaison au passage de la ligne d'arrivée. Le gyroscope doit en principe être calibré initialement pour pouvoir déterminer une différence d'orientation, c'est-à-dire un angle d'inclinaison par rapport à une direction verticale ou horizontale.

Le module à transpondeur est placé sur une partie haute du corps de l'athlète en particulier dans le dossard à numéro. Une fois calibré et activé, le gyroscope détermine l'inclinaison de l'athlète au moment du passage de la ligne d'arrivée pour pouvoir corriger le temps d'arrivée non officiel déterminé et fourni par le module à transpondeur par rapport au temps officiel déterminé avec l'image photofinish. Grâce au signal de données transmis par le module à transpondeur, un classement correct non officiel des athlètes au terme de leur course peut être immédiatement fourni à l'arrivée d'une course sur piste, par exemple une course de sprint.

A cet effet, l'invention concerne aussi un procédé de mesure avec correction du temps d'arrivée d'un athlète dans une course à pieds pour la mise en action du système de mesure, et qui comprend les caractéristiques définies dans la revendication indépendante 10.

Des étapes particulières du procédé de mesure sont définies dans les revendications dépendantes 11 à 14.

### Brève description des dessins

Les buts, avantages et caractéristiques du système de mesure avec correction du temps d'arrivée d'un athlète dans une course à pieds, et le procédé de mise en action du système de mesure apparaîtront mieux dans la description suivante d'au moins une forme d'exécution non limitative illustrée par les dessins sur lesquels :
la figure 1 représente schématiquement les principaux éléments d'un système de mesure avec correction du temps d'arrivée d'un athlète dans une course à pieds selon l'invention,
les figures 2a à 2c représentent de manière schématique un athlète, qui est muni d'un module à transpondeur montrant une vue de son passage d'une ligne d'arrivée en position droite et deux vues avec la partie haute du corps pliée au passage de la ligne d'arrivée, et
la figure 3 représente de manière schématique un athlète, qui est muni d'un module à transpondeur en position de départ sur un starting-block.

### Description détaillée de l'invention

Dans la description suivante, tous les éléments du système de mesure avec correction du temps d'arrivée d'un athlète dans une course à pieds, qui sont bien connus de l'homme du métier dans ce domaine technique, ne seront relatés que de manière simplifiée. La course à pieds peut être effectuée sur piste ou sur route ou en forêt ou en montagne.

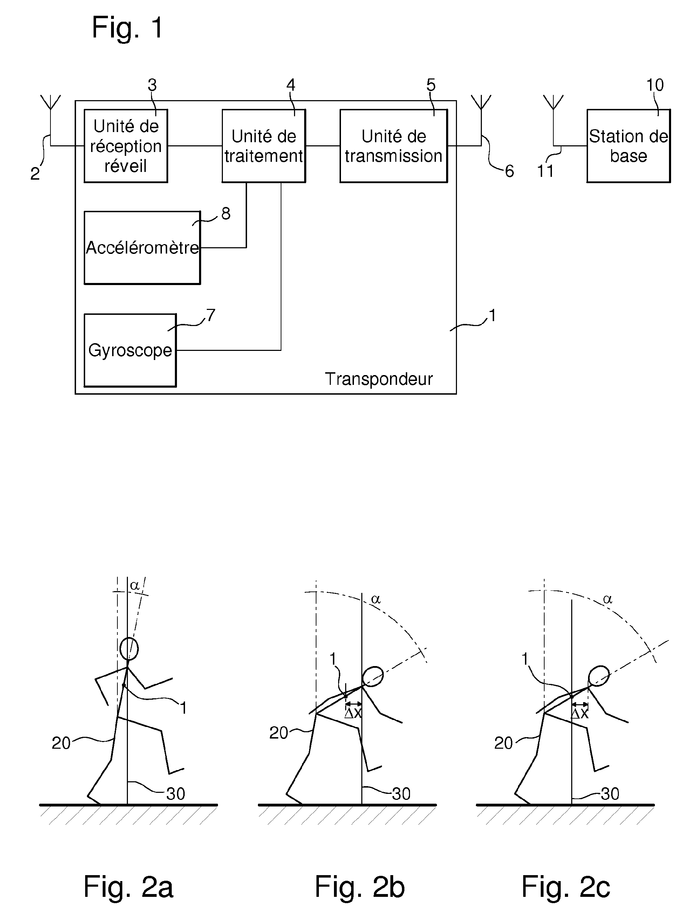

La figure 1 représente schématiquement les principaux éléments, qui composent un système de mesure avec correction du temps d'arrivée d'un athlète dans une course sur piste ou sur route. Pour ce faire, le système comprend un ou plusieurs modules à transpondeur 1 et au moins une station de base 10 pour la communication de données et/ou de mesures et/ou de commandes entre les modules à transpondeur 1 et la station de base 10. Chaque module à transpondeur 1 pour la compétition est disposé sur une partie du corps d'un athlète, par exemple dans un dossard à numéro, et est donc personnalisé ou adapté à l'athlète, qui le porte. De préférence, le module à transpondeur 1 est disposé sur une partie haute du corps de l'athlète, tel que le thorax. Comme expliqué ci-après, ledit module à transpondeur est disposé au niveau du centre gravité, tel que le thorax. Ceci permet de détecter la rotation du haut du corps de l'athlète notamment au moment de passer la ligne d'arrivée pour corriger le temps de course non officiel de l'athlète.

De préférence au niveau de la ligne d'arrivée, il est prévu une boucle antenne au sol ou une antenne de côté alignée avec la ligne d'arrivée pour pouvoir transmettre un signal de champ magnétique. Ce signal de champ magnétique peut être stationnaire ou avec modulation de synchronisation par période déterminée. Le module à transpondeur 1 de chaque athlète en course peut détecter le passage de la ligne d'arrivée en mesurant une ou plusieurs intensités du champ magnétique à proximité de l'antenne d'arrivée. Une prédiction du temps d'arrivée par le module à transpondeur 1 peut aussi être déterminée sur cette base. Une mesure du temps peut aussi être effectuée dans le module à transpondeur 1 ou par transmission de signaux de mesure d'intensités du champ capté à la station de base 10. Il est également possible que le module à transpondeur 1 soit réveillé et/ou synchronisé par une autre antenne au départ ou en différents points de la piste de course.

Le module à transpondeur 1 peut être du type actif avec une batterie ou cellule solaire ou autre source d'énergie intégrée dans le module ou du type passif en étant alimenté par la réception d'un signal d'interrogation ou de réveil traditionnel. Cependant avec l'utilisation d'un ou plusieurs capteurs de mouvement dans le module à transpondeur, il est nécessaire d'avoir un module à transpondeur du type actif.

Le module à transpondeur 1 comprend une unité de réception sans fil de signaux 3 pour recevoir par une antenne 2 des signaux de données ou commandes provenant d'une station de base 10 ou d'un émetteur disposé dans un starting-block du système de mesure ou le long d'un tracé ou de la piste de course ou également au niveau de l'arrivée de la course. De préférence, les signaux reçus par l'antenne 2 liée à l'unité de réception 3 sont des signaux permettant le réveil ou la synchronisation du module à transpondeur 1, qui peut être dans un état de repos avant la réception de tels signaux. Ces signaux de synchronisation sont générés, comme indiqué ci-dessus, par la station de base 10 ou par un émetteur du starting-block ou le long de la piste de course, après le signalement de préparation de départ d'une course d'athlétisme notamment ou directement au moment du coup de pistolet de départ.

Il est à noter que le module à transpondeur 1 peut aussi ne pas comprendre d'unité de réception 3, afin de n'effectuer que des mesures par au moins un capteur de mouvement et transmettre l'information à la station de base pour la correction d'un temps d'arrivée.

Le module à transpondeur 1 comprend encore une unité de traitement 4, qui peut être une machine d'état, un processeur ou un microcontrôleur, pour la gestion de toutes les données ou commandes ou mesures à recevoir ou à transmettre. Une synchronisation de ladite unité de traitement peut également être opérée dès le départ de course, si elle comporte une base de temps pour permettre la détermination d'un temps de course. La synchronisation du module à transpondeur 1 peut également être effectuée en différents points de la course ou à proximité de la ligne d'arrivée. L'unité de traitement 4 reçoit les données ou commandes mises en forme dans l'unité de réception 3 de manière également à réveiller tous les composants, qui composent le module à transpondeur 1. L'unité de traitement 4 est encore reliée à une unité de transmission de signaux 5 par une antenne 6 à destination de la station de base 10. La station de base 10 peut être un système de chronométrage de la course et comprend une antenne 11 de transmission ou réception de signaux.

Le module à transpondeur 1 comprend encore au moins un capteur de mouvement 7 lié à l'unité de traitement 4 pour fournir des signaux de mesure en continu ou par intermittence à l'unité de traitement 4 une fois que le module à transpondeur est réveillé et/ou synchronisé. Le module à transpondeur 1 peut comprendre comme capteur de mouvement au moins un gyromètre ou gyroscope 7. De préférence, le gyroscope 7 permet de déterminer une vitesse de rotation et un angle de rotation de la partie haute du corps de l'athlète pour déterminer un angle α de la partie haute du corps par exemple par rapport à une direction verticale ou perpendiculaire à la piste. Cette détermination du gyroscope n'est évidemment possible, que si l'orientation initiale est connue. Les signaux de mesure sont fournis directement à l'unité de traitement 4. L'angle d'inclinaison α peut aussi être déterminé par rapport à une direction horizontale.

Il est à noter que le capteur de mouvement du module à transpondeur 1 peut être également un ensemble, qui comprend un accéléromètre triaxial 8, un gyroscope triaxial 7 et un capteur magnétique triaxial non représenté. Cet ensemble définit un capteur inertiel à 9 axes.

Le module à transpondeur 1 peut encore comprendre un autre capteur de mouvement, qui est un accéléromètre 8 lié aussi à l'unité de traitement 4. Cet accéléromètre 8 peut mesurer l'accélération d'un athlète au moment du départ de la course et des cadences de foulées durant toute la course et jusqu'à l'arrivée. De plus, le gyroscope 7 peut être triaxial, ainsi que l'accéléromètre 8 de manière à opérer des mesures dans les trois directions x, y, z.

Dans le module à transpondeur 1, les signaux de mesure du gyroscope 7 et de l'accéléromètre 8 ou d'autres types de capteurs sont échantillonnés par l'unité de traitement 4 ou directement par le capteur lui-même avant une transmission à l'unité de traitement 4. Les signaux de mesure peuvent être transmis directement à la station de base 10 en utilisant l'unité de transmission sans fil 5. Toutefois, les signaux de mesure peuvent être améliorés notamment après filtrage et ensuite mémorisés et/ou envoyés par la suite à la station de base 10. Il peut aussi être prévu de traiter les données des différents capteurs et tout événement de détection. Il peut encore être prévu de traiter des caractéristiques de mouvement extraites, telles que la fréquence des pas et transmettre cette information à la station de base 10 en plus des données proprement dites de l'accéléromètre 8 et du gyroscope 7.

Il est encore à noter que les signaux reçus par l'antenne 2 liée à l'unité de réception 3 peuvent être des signaux à basse fréquence de l'ordre de 125 kHz, alors que les signaux transmis par l'antenne 6 liée à l'unité de transmission 5 peuvent être des signaux à fréquence relativement élevée, par exemple des signaux HF ou UHF à fréquence supérieure à 300 MHz. Toutefois, il peut être concevable d'avoir un module à transpondeur 1 avec une seule antenne de réception et d'émission commutable pour la réception ou l'émission de signaux de données. Dans ce cas de figure, il est préféré avoir une réception d'au moins un signal de réveil et une émission de signaux de données à une fréquence porteuse similaire avec une modulation des données transmises FSK, BPSK, QPSK ou ON-OFF Keying.

Comme il est possible de le visionner aux figures 2a à 2c, un athlète 20 peut passer la ligne d'arrivée 30 en position droite, c'est-à-dire avec un faible angle α d'inclinaison de la partie haute du corps par rapport à la verticale. Le module à transpondeur 1 passe donc en même temps que la partie haute du corps comme montré à la figure 2a. Si un athlète 20 se plie vers l'avant, lorsqu'il passe la ligne d'arrivée 30, le module à transpondeur 1 est encore en retrait d'une distance Δx de la ligne d'arrivée 30 comme montré à la figure 2b. Finalement lorsque le module à transpondeur 1 passe sur la ligne d'arrivée comme montré à la figure 2c et est détecté par la station de base du système de mesure de chronométrage, il obtient une estampille temporelle. Cette estampille temporelle est Δx/v trop tard en comparaison du temps officiel final de l'athlète 20, où v est la vitesse de l'athlète. Par exemple pour une course de sprint sur 100 m, la vitesse de l'athlète 20 est voisine de 12 m/s en passant la ligne d'arrivée 30.

En ayant une estimation de l'inclinaison α de la partie haute du corps de l'athlète 20, la vitesse v et la position moyenne du module à transpondeur 1 sur la partie haute du corps, il est possible de fournir par approximation l'erreur de détection et la corriger. A titre d'exemple, si le module à transpondeur 1 est placé à une distance d, par exemple à 25 cm en dessous des épaules de l'athlète 20, il peut être mesuré l'inclinaison α par le gyroscope du module à transpondeur 1 de la partie haute du corps d'un angle α de 66° comme montré aux figures 2b et 2c, avec une vitesse d'une course de sprint à 12 m/s. Cette vitesse de course peut être calculée de préférence dans le module à transpondeur 1, voire dans la station de base 10, au moment du passage de la ligne d'arrivée 30 par les mesures des capteurs de mouvement 7, 8.

La distance d peut aussi être estimée sur la base de paramètres anthropométriques de chaque athlète. Cette distance d peut ainsi être une fonction de la hauteur de l'athlète. La distance d estimée peut aussi être directement fournie avec le fabricant de chaque dossard de course.

Le temps de course pris par le module à transpondeur 1 d'un athlète peut être égal à 9.598 s. L'erreur sur la distance devient Δx = (25 cm)·sin(α) = 22.8 cm et l'erreur temporelle Δt = Δx/v = 0.019 s. Ainsi le temps d'arrivée correct serait alors à 9.579 s, qui est arrondi selon les règles de l'athlétisme à 9.58 s. Cela correspond au temps officiel de course à communiquer par exemple aux spectateurs de ladite course de manière plus rapide que la détermination correcte par l'image photofinish du temps officiel de course de chaque athlète 20.

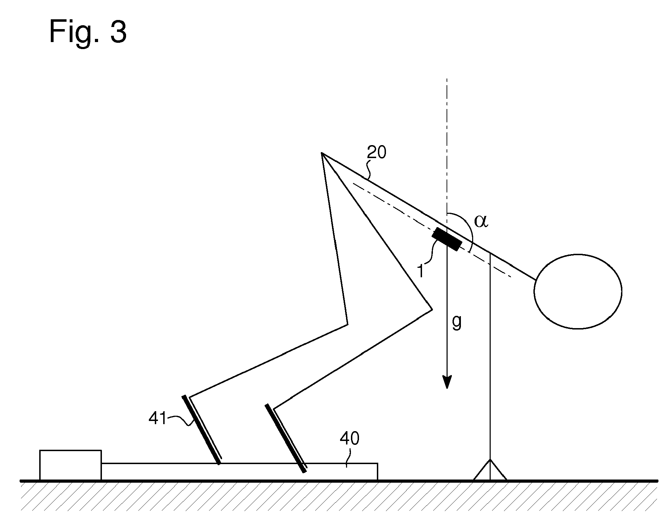

Comme représenté à la figure 3 au moment du départ, un athlète 20, qui est muni d'un module à transpondeur 1, est accroupi et en appui par ses deux pieds contre deux plots d'appui 41 d'un starting-block 40, qui est disposé et fixé sur le sol de la piste. De préférence, le capteur de mouvement peut être un capteur inertiel à 9 axes. Pour faire une estimation de l'inclinaison α de la partie haute du corps sur la base des signaux du gyroscope, il est nécessaire de connaître l'angle initial par exemple au début de la course. Cette angle α peut être obtenu en mesurant le vecteur du centre de gravité g par l'intermédiaire d'un accéléromètre triaxial. Cette angle initial est mémorisé dans le module à transpondeur, qui doit être activé pour cette mesure au moins juste avant le départ. L'angle initial est donc mémorisé durant la phase après l'annonce de préparation au départ et avant le coup de pistolet de départ de la course. Une fois que l'angle initial est connu, l'inclinaison de la partie haute du corps peut être calculée par des opérations de rotation sur la base des signaux du gyroscope durant la course et principalement à l'arrivée.

Il est à noter qu'il est nécessaire d'avoir une condition initiale pour permettre de déterminer l'angle d'inclinaison α. Le module à transpondeur avec le capteur de mouvement doit être calibré pour pouvoir effectué cette mesure de l'angle d'inclinaison lors du passage de la ligne d'arrivée. Cette condition initiale peut être définie de préférence avant le départ de la course, quand l'athlète reste prêt comme montré à la figure 3, mais peut aussi être estimée durant la course avant le passage de la ligne d'arrivée.

Pour le procédé de mesure d'un temps de course pour la mise en action du système de mesure, il peut être prévu de mesurer l'angle de la partie haute du corps d'un athlète en course dans le module à transpondeur. Ce module peut être activé dès le départ ou dans des positions intermédiaires entre le départ et la ligne d'arrivée, ou à l'arrivée. De préférence le module à transpondeur est activé dès le départ de la course. Le capteur de mouvement, qui peut comprendre au moins-le gyroscope, est prévu pour déterminer un angle d'inclinaison de la partie haute du corps de l'athlète, qui porte le module à transpondeur. Une orientation initiale du module à transpondeur peut être définie et mémorisée juste avant le départ de la course. Ainsi une fois que le module à transpondeur s'approche de la ligne d'arrivée et par exemple en détectant des signaux d'interrogation à basse fréquence d'une antenne d'arrivée, une détermination de l'angle d'inclinaison et l'écart temporel peuvent être calculés directement dans ledit module à transpondeur. Une fois calculés, il peut y avoir une transmission de signaux de données ou commandes à destination de la station de base pour définir un temps de course corrigé. Cependant, il peut être aussi prévu qu'uniquement l'angle d'inclinaison mesuré dans le module à transpondeur soit transmis avec un calcul de l'écart temporel effectué directement après le passage de la ligne d'arrivée par la station de base.

Il est à noter que le module à transpondeur activé peut également transmettre toutes les données de mesure effectuées par ses capteurs à la station de base, qui dans ce cas détermine et calcule l'écart temporel et la correction du temps non officiel de chaque athlète.

A partir de la description qui vient d'être faite, plusieurs variantes du système et procédé de mesure avec correction du temps d'arrivée d'un athlète dans une course peuvent être conçues par l'homme du métier sans sortir du cadre de l'invention définie par les revendications. Il peut être prévu d'avoir plusieurs modules à transpondeur sur une partie du corps de l'athlète pour une détermination de l'angle d'inclinaison au passage de la ligne d'arrivée et la correction du temps d'arrivée non officiel.

## Revendications

1. Système de mesure avec correction d'un temps d'arrivée d'au moins un athlète (20) dans une course à pieds, le système de mesure comprenant au moins un module à transpondeur (1) personnalisé et placé sur un athlète et une station de base (10), ledit module à transpondeur (1) comprenant une unité de traitement (4) de données, de mesures ou de commandes, une unité de transmission (5) de signaux de données et/ou de mesures et/ou de commandes, et au moins un capteur de mouvement (7) pour fournir des signaux de mesure à l'unité de traitement (4),
**caractérisé en ce que** le capteur de mouvement est configuré de telle manière à déterminer un angle d'inclinaison α du corps de l'athlète par rapport à une direction verticale ou horizontale lors du passage de la ligne d'arrivée (30) pour une correction d'un temps d'arrivée de course.

2. Système de mesure selon la revendication 1, **caractérisé en ce que** le capteur de mouvement est un gyroscope (7) relié à l'unité de traitement (4) dans le module à transpondeur (1).

3. Système de mesure selon la revendication 2, **caractérisé en ce que** le module à transpondeur (1) comprend encore un autre capteur de mouvement, qui est un accéléromètre (8) relié à l'unité de traitement (4) pour la détection d'une variation de mouvement dudit module ou d'un niveau de vibrations dudit module.

4. Système de mesure selon la revendication 3, **caractérisé en ce que** le gyroscope (7) est un gyroscope triaxial, et **en ce que** l'accéléromètre (8) est un accéléromètre triaxial.

5. Système de mesure selon la revendication 1, **caractérisé en ce que** le capteur de mouvement est un capteur inertiel à 9 axes, qui comprend un ensemble avec un accéléromètre triaxial (8), un gyroscope triaxial (7) et un capteur magnétique triaxial.

6. Système de mesure selon la revendication 2, pour lequel le module à transpondeur (1) est disposé sur une partie haute du corps de l'athlète (20), **caractérisé en ce que** le gyroscope (7) est configuré pour être calibré au début d'une course et pour pouvoir déterminer un angle d'inclinaison α du corps de l'athlète au passage de la ligne d'arrivée, afin de calculer le temps de course de l'athlète.

7. Système de mesure selon la revendication 6, **caractérisé en ce que** l'unité de traitement (4) calcule une erreur temporelle Δt d'un temps de course de l'athlète (20) sur la base de l'angle d'inclinaison α par rapport à une direction verticale, de la position d du module à transpondeur (1) par rapport aux épaules de l'athlète et de la vitesse de l'athlète v à l'arrivée, l'erreur temporelle étant Δt = Δx/v, où Δx = d·sin(α).

8. Système de mesure selon la revendication 7, **caractérisé en ce que** l'unité de traitement (4) détermine un temps de course de l'athlète corrigé dans le module à transpondeur (1) pour une transmission du temps de course correct de l'athlète à la station de base (10).

9. Système de mesure selon l'une des revendications précédentes, **caractérisé en ce que** le module à transpondeur comprend en outre une unité de réception sans fil de signaux (3) pour recevoir par une antenne (2) des signaux de données ou commandes provenant de la station de base (10) ou d'un émetteur disposé au départ de la course ou le long d'un tracé de course.

10. Procédé de mesure avec correction du temps d'arrivée d'un athlète dans une course à pieds pour la mise en action du système de mesure selon l'une des revendications précédentes, pour lequel le système de mesure comprend au moins un module à transpondeur (1) personnalisé et placé sur un athlète et une station de base (10), ledit module à transpondeur (1) comprenant une unité de traitement (4) de données, de mesures ou de commandes, une unité de transmission (5) de signaux de données et/ou de mesures et/ou de commandes, et au moins un capteur de mouvement (7) pour fournir des signaux de mesure à l'unité de traitement (4), **caractérisé en ce que** le procédé comprend les étapes de :
- mesurer un angle d'inclinaison α de l'athlète (20) au moyen d'un capteur de mouvement du module à transpondeur (1) activé au moment de passer la ligne d'arrivée (30),
- corriger un temps d'arrivée de course de l'athlète sur la base de la mesure de l'angle d'inclinaison α dans le module à transpondeur (1) ou dans la station de base (10).

11. Procédé de mesure selon la revendication 10, pour lequel le capteur de mouvement est un gyroscope (7) relié à l'unité de traitement (4) dans le module à transpondeur (1), **caractérisé en ce qu'**une fois activé, une mesure d'accélération de l'athlète est effectuée par un autre capteur de mouvement, qui est un accéléromètre (8) du module à transpondeur (1) de manière à détecter une variation de mouvement dudit module ou d'un niveau de vibrations dudit module.

12. Procédé de mesure selon l'une des revendications 10 et 11, **caractérisé en ce que** le module à transpondeur (1) est synchronisé dès le départ de la course ou en différents points de la course ou à proximité de la ligne d'arrivée par un signal d'interrogation ou de réveil.

13. Procédé de mesure selon la revendication 12, **caractérisé en ce qu'**une fois activé et synchronisé, le module à transpondeur (1) détermine un temps de course corrigé au passage de la ligne d'arrivée sur la base de mesures d'intensités d'un signal de champ magnétique d'une antenne à proximité de la ligne d'arrivée (30) et capté par une unité de réception (3), et **en ce que** le temps d'arrivée de course corrigé est transmis du module à transpondeur (1) à la station de base (10).

14. Procédé de mesure selon l'une des revendications 11 à 13, **caractérisé en ce qu'**un calcul d'une erreur temporelle Δt d'un temps d'arrivée de course de l'athlète (20) est effectué dans l'unité de traitement (4) du module à transpondeur sur la base de l'angle d'inclinaison α par rapport à une direction verticale, de la position d du module à transpondeur (1) par rapport aux épaules de l'athlète et de la vitesse de l'athlète v à l'arrivée, l'erreur temporelle étant Δt = Δx/v, où Δx = d·sin(α).
